# EUROPEAN PATENT APPLICATION

(11) **EP 2 316 461 A1**
(43) Date of publication of application: **04.05.2011**
(21) Application number: 10160096.3
(22) Date of filing: 15.04.2010
(51) Int. Cl.: A61K 31/785, A61K 31/047

(54) **Composition for treating dermal tissue**

(30) Priority: 15.04.2009 US 423929
(71) Applicant: Tyco Healthcare Group LP, Mansfield, MA 02048 (US)
(72) Inventor: Copp-Howland, Warren W., Chicopee, MA 01013 (US); Garstka, Erick, Westfield, MA 01085 (US); Hyatt, Chris, South Hadley, MA 01075 (US); Tremblay, Kathleen, Westfield, MA 01085 (US); Selvitelli, David, Suffield, CT 06078 (US)
(74) Representative: Jones, Nicholas Andrew

(57) **Abstract**

The present disclosure provides gel compositions useful for treating the skin. The gel compositions are hydrogels that include from substantially 33 wt% to substantially 68 wt% of a humectant or a mixture of humectants; substantially 0 wt% to substantially 8 wt% of an electrolyte or mixture of electrolytes; from substantially 2 wt% to substantially 20 wt% of water; from substantially 18 wt% to substantially 45 wt% of a copolymer. The copolymer includes, in polymerized form, from substantially 80 mol% to substantially 95 mol% of a first monomer, which is a mixture of acrylic acid and a salt thereof, from substantially 5 mol% to 20 mol% of a second monomer, which can be a salt of 2-acrylamido-2-methylpropane sulfonic acid, and, optionally a crosslinking agent. In embodiments, the gel composition has a pH of substantially 7.0 or less. The dermal patches of the present disclosure may be utilized in skin care, and may include the application of the patch, its removal, and re-application to tissue.

## Description

### BACKGROUND

### Technical Field

The present disclosure relates to external mammalian skin care. In embodiments, the disclosure relates to gels which may be utilized to treat dry, cracked, and/or irritated skin. The gel of the present disclosure may provide a moist environment for healing. In some embodiments, the gel may be utilized with a substrate to provide further protection to dry, cracked and/or irritated skin. For example, the gel may be utilized with breast care pads for use by nursing mothers to prevent unwanted leakage of breast milk, while at the same time providing a moist environment to protect the mammilla from drying out.

### Background of Related Art

Skin is subject to insults by many factors. Extrinsic factors that can adversely affect the skin include ultraviolet radiation (e.g., from sun exposure), environmental pollution, wind, heat, low humidity, harsh surfactants, abrasives, and the like, as well as wounds caused by cuts, scratches, punctures, and the like. Such insults can result in both wounds and skin that is dry, cracked, irritated, and the like. Another example of skin irritation includes mothers attempting to breast feed their babies, who may experience sore and painful nipples at some point during post partum care. For many women, this can lead to tenderness, dryness, cracking, leaking, bleeding nipples, and even breast infections. Current commercially available products may address one or two of these issues, but, do not address all of these issues. Moreover, there is no satisfactory treatment that provides immediate relief for all of these conditions.
Improved skin care compositions thus remain desirable.

### SUMMARY

The present disclosure is directed to a means for providing care to dermal tissue. In embodiments, a gel may be utilized to treat irritated or damaged dermal tissue, including dried and/or cracked dermal tissue. In embodiments, the gel may be used alone. In other embodiments, the gel may be applied to a substrate and then applied to the irritated or damaged tissue. For example, in embodiments, the present disclosure includes a dermal patch including a gel composition and a substrate. The substrate may have several layers, such as a fluid impermeable outer cover, an absorbent layer, a wicking layer, and an inner liner.

The gel composition includes, in embodiments, from about 33 wt% to about 68 wt% of a humectant or a mixture of humectants; optionally, from about 0 wt% to about 8 wt% of an electrolyte or mixture of electrolytes; from about 2 wt% to about 20 wt% of water; from about 18 wt% to about 45 wt% of a copolymer including, in polymerized form, from about 80 mol% to about 95 mol% of a first monomer, in which the first monomer is a mixture of acrylic acid and one or more salts thereof, and from about 5 mol% to 20 mol% of a second monomer, in which the second monomer is one or more monomers selected from CH₂=C(O)XR, in which X is O or NH and R is an unsubstituted or substituted alkyl group of from about 1 to about 5 carbon atoms; and the composition has a pH of about 7 or less.

The gel composition is either not crosslinked or is crosslinked by a crosslinking agent. The second monomer may be a salt of 2-acrylamido-2-methylpropane sulfonic acid, and the pH of the composition is from about 3 to about 6.5. In some embodiments of the gel composition, the first monomer is a mixture of acrylic acid and sodium acrylate, and the second monomer is sodium 2-acrylamido-2-methylpropane sulfonate. In some embodiments of the gel composition, the composition includes from about 55 wt% to about 65 wt% of the humectant or the mixture of humectants and from about 20 wt% to about 40 wt% of the copolymer, and the copolymer includes, in polymerized form, from about 85 mol% to about 95 mol% of the first monomer and from about 5 mol% to 15 mol% of the second monomer. In some embodiments of the gel composition, the humectant is glycerol, sorbitol, or a mixture thereof, and the electrolyte is sodium chloride. In some embodiments of the gel composition, the composition includes from about 0.01 wt% to about 1.0 wt% of a crosslinking agent or mixture of crosslinking agents.

Methods for treating irritated or damaged dermal tissue with the gels are provided. In embodiments, a method of the present disclosure includes providing a gel composition including: from about 33 wt% to about 68 wt% of a humectant or a mixture of humectants; optionally, from about 0 wt% to about 8 wt% of an electrolyte or mixture of electrolytes; from about 2 wt% to about 20 wt% of water; from about 18 wt% to about 45 wt% of a copolymer comprising, in polymerized form, from about 80 mol% to about 95 mol% of a first monomer, in which the first monomer is a mixture of acrylic acid and one or more salts thereof, and from about 5 mol% to about 20 mol% of a second monomer, in which the second monomer is one or more monomers selected from CH₂=C(O)XR, in which X is O or NH and R is an unsubstituted or substituted alkyl group of from about 1 to about 5 carbon atoms; in which the composition has a pH of about 7.0 or less; and applying the gel to the irritated or damaged dermal tissue.

In other embodiments, methods of the present disclosure may include providing a gel composition including: from about 33 wt% to about 68 wt% of a humectant or a mixture of humectants; optionally, from about 0 wt% to about 8 wt% of an electrolyte or mixture of electrolytes; from about 2 wt% to about 20 wt% of water; from about 18 wt% to about 45 wt% of a copolymer comprising, in polymerized form, from about 80 mol% to about 95 mol% of a first monomer, in which the first monomer is a mixture of acrylic acid and one or more salts thereof, and from about 5 mol% to about 20 mol% of a second monomer, in which the second monomer is one or more monomers selected from CH₂=C(O)XR, in which X is O or NH and R is an unsubstituted or substituted alkyl group of from about 1 to about 5 carbon atoms; in which the composition has a pH of about 7.0 or less; and providing a substrate cover configured to retain the gel composition against the dermal tissue and prevent the gel composition from contacting another surface.

A dermal patch with such a gel for treating tissue is also provided. In embodiments, a dermal patch may include a substrate, and the gel composition described above applied to the substrate for positioning adjacent dermal tissue. In some embodiments, the dermal patch includes a wicking layer, an absorbent layer, and a release liner releasably attached to the gel composition.

In one aspect, the dermal patch may be a cup shaped to fit comfortably over the breast of a mammal for post-partum breast care. In another aspect, the dermal patch may be applied to the skin to prevent potential chaffing, absorb fluid leakage, and provide a moist environment for dry or cracked skin tissue.

In another aspect, the dermal patch provides a cooling sensation to provide relief to the patient. In one aspect, the dermal patch can be reused several times and can be stored by placing the dermal patch on a release liner. A method for treating areola tissue with the dermal patch is also provided.

In embodiments, a dermal patch for application to irritated or damaged dermal tissue may include a substrate; and a gel composition for placement adjacent the dermal tissue applied to the substrate, the gel composition including: from about 33 wt% to about 68 wt% of a humectant or a mixture of humectants; optionally, from about 0 wt% to about 8 wt% of an electrolyte or mixture of electrolytes; from about 2 wt% to about 20 wt% of water; from about 18 wt% to about 45 wt% of a copolymer comprising, in polymerized form, from about 80 mol% to about 95 mol% of a first monomer, in which the first monomer is a mixture of acrylic acid and one or more salts thereof, and from about 5 mol% to about 20 mol% of a second monomer, in which the second monomer is one or more monomers selected from CH₂=C(O)XR, in which X is O or NH and R is an unsubstituted or substituted alkyl group of from about 1 to about 5 carbon atoms; and in which the composition has a pH of about 7.0 or less.

In yet other embodiments, the present disclosure provides gel compositions including from about 33 wt% to about 68 wt% of a humectant or a mixture of humectants; from about 2 wt% to about 20 wt% of water; from about 18 wt% to about 45 wt% of a copolymer comprising, in polymerized form, from about 80 mol% to about 95 mol% of a first monomer, in which the first monomer is a mixture of acrylic acid and one or more salts thereof, and from about 5 mol% to about 20 mol% of a second monomer, in which the second monomer is one or more monomers selected from CH₂=C(O)XR, in which X is O or NH and R is an unsubstituted or substituted alkyl group of from about 1 to about 5 carbon atoms; and in which the composition has a pH of about 7.0 or less.

### BRIEF DESCRIPTION OF THE DRAWINGS

Embodiments of the present disclosure are described herein with reference to the accompanying drawings, wherein:
FIG. 1 is a top plan view of a dermal patch including the gel composition according to the present disclosure;
FIG. 2 is a cross-sectional view of a dermal patch;
FIG. 3 is a top plan view of a dermal patch including a contoured profile for positioning over the patient's breast in accordance with another embodiment of the present disclosure;
FIG. 4 is a cross-sectional view of a dermal patch of Figure 3 positioned over a human female breast; and
FIG. 5 is a perspective view of the dermal patch of Figures 3-4.

Other features and advantages of the present disclosure will become apparent from the following detailed description, taken in conjunction with the accompanying drawings, which illustrate, by way of example, the principles of the present disclosure.

### DETAILED DESCRIPTION OF THE EMBODIMENTS

Unless the context indicates otherwise, in the specification and claims, the terms first monomer, second monomer, humectant, optional electrolyte, polymerization initiator, polymerization inhibitor, crosslinking agent, neutralizer, and similar terms also include mixtures of such materials. Unless otherwise specified, all percentages are percentages by weight and all temperatures are in degrees Centigrade (degrees Celsius).

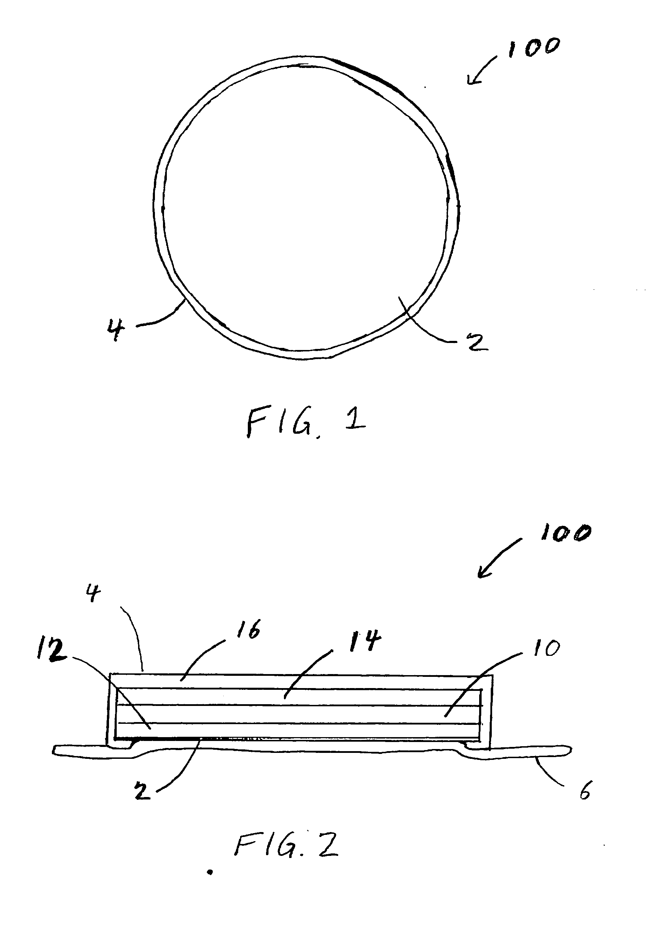

Referring now to Figures 1-2, a dermal patch in accordance with the present disclosure will be discussed in detail. Dermal patch 100 includes a substrate which can provide protection from external elements and creates an environment promoting healing of irritated or damaged skin. In some embodiments, the skin may encompass the breast and/or nipple, including the areola. Dermal patch 100 includes a gel composition 2 applied to a substrate 4, and a release liner 6 covering the gel composition 2. A suitable gel composition 2 is disclosed in commonly assigned U.S. Patent Application Publication No. 2007/0282188, the entire disclosure of which is incorporated herein by reference. Some of the specifics of the gel composition will be discussed hereinbelow.

The substrate 4 of the dermal patch 100 may be made from any suitable material, including but not limited to, wovens, nonwovens, films, cotton cloths, polyesters, polyethylenes, polyurethanes, treated paper materials, plastics, combinations thereof, and the like. The substrate 4 provides a flexible base for applying the gel composition 2 and for retaining the gel composition 2 before, during, and after use. A flexible substrate may be especially useful for application to the skin, as it may be able to bend, twist and flex with, and conform to, the skin to which it is applied with a gel of the present disclosure. This may thus minimized interference with the patient's daily routine and activities. Substrate 4 may also prevent the gel composition 2 from sticking to fabrics, such as clothing, placed over the gel composition 2.

The substrate 4 may include one or more layers. Where substrate 4 includes multiple layers, one of the layers may be a wicking layer 10 that assists in drawing fluids or exudates away from the skin. In other embodiments, one of the layers may be an absorbent layer 14, such as but not limited to cotton or cellulose, to capture fluids. An inner liner 12 may also be included to protect the various layers and provide an outer permeable layer. A fluid impermeable outer cover 16 may also be used to limit the transfer of the gel composition 2 to surrounding clothing and to contain the gel composition 2 within a predetermined boundary.

The dermal patch 100 may include any combination of one or more of these layers. In embodiments, the gel composition 2 may be located between one or more of these layers. Further, a release liner 6 may be placed over the gel composition 2 to contain the gel composition 2 between the substrate 4 and the release liner 6 during storage. The release liner 6 may be made of such material as, but not limited to, a release paper, or a film of waxed or coated plastic, e.g. silicone coated polyethylene terephthalate film. The release liner 6 is removably applied over the gel composition 2.

The gel composition 2 has sufficient adhesive properties to hold the gel composition 2 against a patient's skin. These adhesive properties are retained during an extended shelf life and during multiple repeated applications of the dermal patch 100. As noted above, the gel composition may be utilized with a substrate to form a dermal patch of the present disclosure or, in alternate embodiments, may be utilized by itself to promote wound healing and treat damaged and irritated skin

In one aspect, the gel composition 2 has properties and characteristics that address issues of tenderness, dryness, cracking, and other treatments of the skin. The gel composition 2 minimizes chaffing, absorbs fluid leakage, and provides a moist environment for dry or cracked skin tissue to heal. The gel composition 2 has re-stick properties that permit the patient to remove and reapply the gel, optionally as part of a dermal patch, over a desired course of treatment while maintaining adhesion to the skin. As noted above, in some embodiments, the gel composition and/or dermal patch may be useful for leaking of the breast associated with post partum breast-feeding and pumping.

In use, the release liner 6 is removed from the dermal patch 100. The substrate 4, with the gel composition 2, is then applied to the skin, e.g., over the breast nipple. The gel composition 2 gently adheres to the skin to provide the aforementioned benefits during application including absorption of leaking fluids, which would otherwise become a source of embarrassment to the patient. The gel composition 2 also provides a cooling sensation to the area of treatment providing additional soothing qualities and relief to the patient. The substrate 4 and the gel composition 2 may be removed after the course of treatment. The release liner 6 may be reapplied to the gel composition 2 for storage of the dermal patch 100.

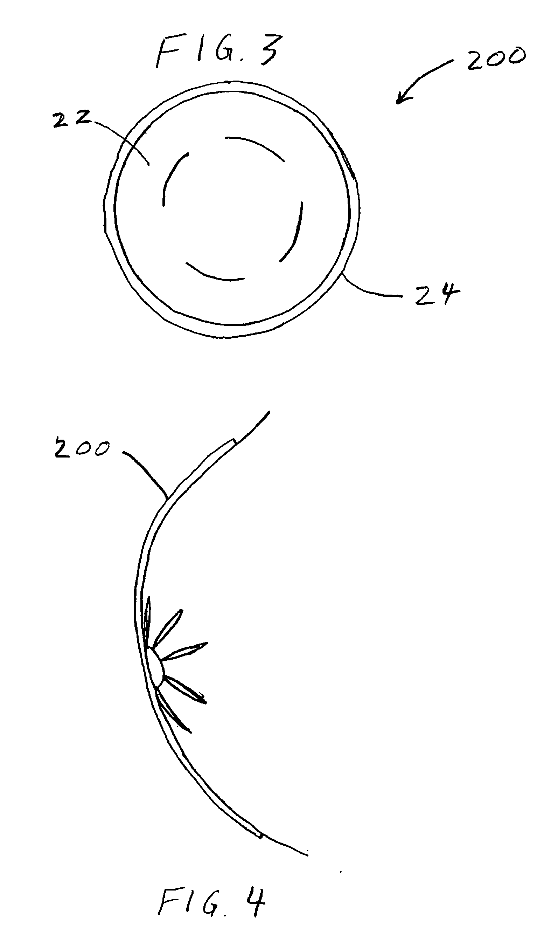

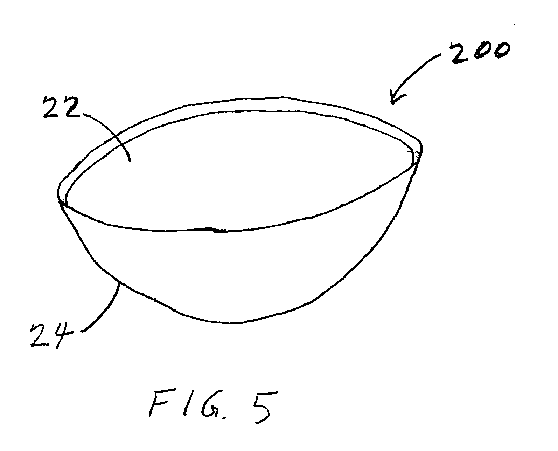

FIGS. 3-5 illustrate another embodiment with a dermal patch 200 having a curved contour or cup shape for application to the breast of the patient. The dermal patch 200 is shaped to fit comfortably over the breast of a mammal for post-partum breast care and includes the gel composition 22 applied to a contoured substrate 24. The dermal patch 200 may have a release liner (not shown).

To use the dermal patch 200, the release liner, similar to release liner 6 in FIG. 2, is removed and the dermal patch 200 is applied to the patient's breast, specifically placing the composition 22 over the nipple. The dermal patch 200 may then be removed and placed on the release liner for storage. The dermal patch 200 may be then reapplied and removed, as required.

The gel composition 2 will now be discussed. The gel composition 2, is a hydrogel and is prepared by polymerizing a pre-gel reaction mixture including a humectant, an optional electrolyte, a first monomer, in which the first monomer is a mixture of acrylic acid and a salt thereof, and a second monomer, in which the second monomer is one or more monomers of the formula CH₂=C(O)XR, in which X is O or NH and R is an unsubstituted or substituted alkyl group of from about 1 to about 5 carbon atoms; polymerization initiator; neutralizer such as sodium hydroxide; water; and optionally, a crosslinking agent, and optionally, a polymerization inhibitor. The pH of the pre-gel reaction mixture, and of the resulting composition, may be about 7.0 or less, in embodiments from about 3.0 to about 6.5, and in other embodiments from about 3.5 to about 5.5.

The pre-gel reaction mixture includes a humectant or a mixture of humectants. As used herein, a "humectant" includes, in embodiments, any material which promotes the retention of moisture. The humectant is a non-volatile, non-toxic, water soluble or water miscible viscous liquid at room temperature. Suitable humectants include polyhydric alcohols such as glycerin, sorbitol, ethylene glycol, propylene glycol, polyethylene glycols such as PEG 400 and PEG 600, poly(propylene glycol), and mixtures thereof. The mixture may include from about 33 wt% to about 68 wt%, in embodiments from about 35 wt% to about 65 wt%, in other embodiments from about 55 wt% to about 60 wt%, of the humectant.

The pre-gel reaction mixture may include an optional electrolyte or a mixture of electrolytes. The electrolyte in some embodiments may be a salt, such as lithium chloride, sodium chloride, potassium chloride, magnesium acetate, ammonium acetate, or a mixture thereof. The mixture includes from about 0 wt% to about 10 wt%, in some embodiments from about 0 wt% to about 8 wt%, in other embodiments from about 2.0 wt% to about 6 wt% of the electrolyte.

The pre-gel reaction mixture includes a monomer mix. The monomer mix includes a first monomer, a second monomer, and, optionally, a cross-linking agent. The first monomer is acrylic acid and a salt thereof, combined as a mixture. The polymer produced by polymerization includes acid acrylate moieties (-CO₂H and/or -CO₂M, in which M is a cation such as sodium ion, potassium ion, lithium ion, ammonium or substituted ammonium ion, etc.) directly attached to the polymer backbone. In embodiments, the first monomer may be a mixture of acrylic acid and sodium acrylate.

The second monomer is one or more monomers such as CH₂=C(O)XR, in which X is O or NH and R is an unsubstituted or substituted alkyl group of from about 1 to about 5 carbon atoms. The polymer produced by polymerization includes groups of the structure -C(O)XR directly attached to the polymer backbone. In some embodiments unsubstituted alkyl groups are methyl, ethyl, *n*-propyl, *n*-butyl, and *n*-pentyl. Suitable substituents that may be present in a substituted alkyl group are halo (such as F, Cl, or Br) cyano, carboxylic acid and salts thereof (*i*.*e*., -CO₂H or -CO₂M, in which M is a cation), phosphate and salts thereof, and sulfonic acid and salts thereof. An example of such a substituted alkyl group is (3-sulfopropyl)acrylic acid ester, potassium salt. A second monomer can be 2-acrylamido-2-methylpropane sulfonic acid (CH₂=CH-CONHC(CH₃)₂-CH₂-SO₃H) and/or a salt thereof. In some embodiments salts are the sodium, lithium, potassium, ammonium, and substituted ammonium salts, and mixtures thereof. In embodiments the second monomer may be sodium 2-acrylamido-2-methylpropane sulfonate.

The monomer mix includes from about 18 wt% to about 45 wt%, in some embodiments from about 20 wt% to 40 wt%, of the pre-gel reaction mixture. Following the polymerization reaction, the resulting composition includes from about 18 wt% to about 45 wt%, in embodiments from about 20 wt% to about 40 wt%, of the copolymer. In the calculation of the percentage composition, acrylic acid and acrylic acid salt or salts are calculated as acrylic acid, and when 2-acrylamido-2-methylpropane sulfonic acid and/or a salt or salts thereof are present, they are calculated as the sodium salt of 2-acrylamido-2-methylpropane sulfonic acid (NaAMPS).

The first monomer makes up from about 80 mol% to about 95 mol%, in some embodiments about 85 mol% to about 92 mol%, of the monomers present in the monomer mix *(i.e.,* the total amount of the first monomer and the second monomer present in the monomer mix). The second monomer makes up from about 5 mol% to about 20 mol%, in some embodiments from about 8 mol% to about 15 mol%, of the monomer mix. In one embodiment the acrylic acid and salt or salts thereof make up about 90 mol% and the second monomer, such as 2-acrylamido-2-methylpropane sulfonic acid and/or salt or salts thereof, make up about 10 mol% of the monomer mix.

In one embodiment, the second monomer is 2-acrylamido-2-methylpropane sulfonic acid sodium salt (NaAMPS) (CH₂=CH-CONHC(CH₃)₂-CH₂-SO₃⁻ M⁺). The first monomer (acrylic acid and salt or salt thereof, calculated as acrylic acid) includes from about 60 wt% to about 85 wt%, in embodiments from about 70 wt% to about 80 wt%, of the total amount of first monomer and the NaAMPS in the monomer mix. The NaAMPS (calculated as NaAMPS) includes from about 15 wt% to about 40 wt%, in embodiments from about 20 wt% to about 30 wt% of the total amount of first monomer and NaAMPS in the monomer mix. In one embodiment, the first monomer includes from about 70 wt% to 75 wt% and the NaAMPS includes from about 25 wt% to 30 wt% of the total amount of first monomer and NaAMPS in the monomer mix.

Optionally, the pre-gel reaction mixture may include an effective amount, in embodiments 1 wt% or less, of a cross-linking agent or mixture of cross-linking agents. An effective amount of cross-linking agent is an amount that produces a composition with the desired physical properties, such as coherence and adhesion. Although the amount required will depend on, for example, the molecular weight of the cross-linking agent, the number of ethylenically unsaturated, free radical polymerizable groups present in the cross-linking agent, the amount of free radical polymerizable monomers present in the monomer mix, when the cross-linking agent is present, the amount of crosslinking agent will equal from about 0.01 wt% to about 1 wt%, in some embodiments from about 0.02 wt% to about 0.08 wt%, of the total weight of the first and second monomers, calculated as described above. The crosslinking agents are free radical polymerizable monomers that include more than one ethylenically unsaturated, free radical polymerizable group. The effective amount of crosslinking agent is soluble in the mixture. Numerous crosslinking agents polymerizable by free-radical initiated polymerization are known to those skilled in the art, Crosslinking agents include, for example, *bis*-acrylamides and methacrylamides, such as N,N'-methylene *bis*-acrylamide; acrylate and methacrylate esters of polyols, such as, ethylene glycol diacrylate and dimethacrylate, diethylene glycol diacrylate and dimethacrylate, trimethylolpropane triacrylate and trimethacrylate, ethoxylated trimethylolpropane triacrylate and trimethacrylate; pentaerythritol triacrylate and trimethacrylate, pentaerythritol tetraacrylate and tetramethacrylate, and polyethylene glycol diacrylates and dimethacrylates, such as the diacrylates and dimethacrylates of polyethylene glycols having a molecular weight of from about 200 to about 600. An especially useful crosslinking agent is N,N'-methylene *bis*-acrylamide [(CH₂=CHCONH)₂CH₂].

The crosslinking-agent may be added to the pre-gel reaction mixture, as, for example, a 1% solution in water. The amount of crosslinking agent is calculated as the amount of the crosslinking agent added, not as the amount of solution containing crosslinking agent added. The water in which the crosslinking agent is dissolved is counted as part of the water present in the mixture.

Although a crosslinking agent may be used to prepare the copolymer, it has been discovered that a composition with the desired properties can be prepared without the use of a cross-linking agent. That is, the resulting copolymer is not crosslinked by a cross-linking monomer (*i.e.,* a copolymer not crosslinked by a crosslinking agent).

The pre-gel reaction mixture includes an effective amount of a polymerization initiator. An effective amount is an amount that produces efficient polymerization of the monomers under the polymerization conditions to produce a composition with the desired properties. Numerous free radical polymerization initiators are within the purview of those skilled in the art. The polymerization initiator may be a single compound or a mixture of compounds. Thermal and/or photo free radical polymerization initiators, for example, may be used. Typical thermal free radical polymerization initiators include azo compounds, such as 2,2-azobisisobutyronitrile (AIBN). Suitable photo free radical polymerization initiators are disclosed in "Photoinitiators for Free-Radical-Initiated Photoimaging Systems," by B. M. Monroe and G. C. Weed, Chem. Rev., 93, 435-448 (1993) and in "Free Radical Polymerization" by K. K. Dietliker, in Chemistry and Technology of UV and EB Formulation for Coatings, Inks, and Paints, P. K. T. Oldring, ed, SITA Technology Ltd., London, 1991, Vol. 3, pp. 59-525. Typical free radical photo polymerization initiators include, for example, 1-hydroxycyclohexylphenyl ketone (HCPK, IRGACURE® 184); 2-hydroxy-2-methyl-1-phenylpropan-1-one (DAROCUR® 1173); 2-hydroxy-1-[4-(2-hydroxyethoxy)phenyl]-2-methyl-1-propan-1-one (IRGACURE®2959), 2,2-dimethoxy-2-phenylacetophenone (benzildimethyl ketal, BDK, IRGACURE®651), and a mixture of 50 wt% benzophenone and 50 wt% of 1-hydroxycyclohexylphenyl ketone (IRGACURE® 500). The pre-gel reaction mixture in some embodiments includes less than about 1.0 wt%, in some embodiments less than about 0.7 wt%, and in some embodiments less than about 0.4 wt%, of the polymerization initiator.

The pre-gel reaction mixture may include a neutralizer. Bases such as hydroxides, amines, Lewis bases, and mixtures thereof may be used as neutralizers. The neutralizer in some embodiments is a base such as ammonium hydroxide, sodium hydroxide, potassium hydroxide, and/or lithium hydroxide. If the acrylic acid and/or the second monomer, such as the 2-acrylamido-2-methylpropane sulfonic acid, are added to the mixture at least partly in the acid form, it may be necessary to added neutralizer to the mixture to neutralize some of the acid so that the pH of the mixture can be from about 3.0 to about 6.5. In one embodiment, all the 2-acrylamido-2-methylpropane sulfonic acid in the pre-gel reaction mixture is neutralized (*i.e.,* present as a salt rather than as the acid), so 2-acrylamido-2-methylpropane sulfonic acid may be added to the pre-gel reaction mixture and neutralized by addition of a neutralizer. Alternatively, a salt or salts of the second monomer, such as the sodium salt, may be added so that the addition of a neutralizer to convert the acid form of the second monomer to a salt is unnecessary. In some embodiments, only part of the acrylic acid is neutralized (i. e., present as a salt). Consequently, if acrylic acid is added, a neutralizer may be necessary to convert part of the acrylic acid to a salt or a mixture of salts. Alternatively, an appropriate mixture of acrylic acid and a salt or a mixture of salts may be added so addition of a neutralizer to convert part of the acrylic acid to a salt or mixture of salts is unnecessary. In some embodiments from about 10 mol% to about 60 mol% of the acrylic acid, in some embodiments from about 25 mol% to about 50 mol% of the acrylic acid, is present in the pre-gel reaction mixture as a salt.

When acrylic acid and NaAMPS are present in the pre-gel reaction mixture, a neutralizer, such as sodium hydroxide, is added. The amount of neutralizer added is less than the amount necessary to neutralize all the acrylic acid in the mixture, so that the resulting mixture has a pH of from about 3.0 to about 6.5. In some embodiments from about 10 mol% to about 60 mol% of the acrylic acid, in some embodiments from about 25 mol% to about 50 mol% of the acrylic acid is neutralized by the neutralizer.

When sodium hydroxide is used as the neutralizer, from about 2 wt% to 8 wt% sodium hydroxide (dry weight) is added to the mixture. The sodium hydroxide may be conveniently added to the mixture as a water solution, such as, for example, an aqueous 50 wt% sodium hydroxide solution.

Water is present in the mixture. The amount of water includes any water present in any of the ingredients and any water added with ingredients that are in water solution, such as the monomers, the crosslinking agent, the neutralizer, the humectant, etc.

As will be apparent to those skilled in the art, when neutralizer is added to neutralize acrylic acid and/or the second monomer, water will be generated by the neutralization reaction. When the water produced by the neutralization reaction, if any, is included, the mixture includes from about 2 wt% to about 20 wt% water. When the water produced by partial neutralization of the acrylic acid is not included, the mixture includes from about 5 wt% to about 18 wt% water.

In addition to the free radical initiator, small amounts of free radical polymerization inhibitors may be present in one or more of the monomers, and/or the crosslinking agent, and/or may be added to the mixture to prevent premature polymerization of the reaction mixture. In some embodiments, free radical polymerization inhibitors include, for example, hydroquinone, 4-methoxyphenol, di-*t-*butyl-*p*-cresol, pyrogallol, *t*-butyl catechol, benzoquinone, 4,4'-thio-bis-(3-methyl-6-*t-*butylphenol), and 2,2'-methylene-bis-(4-methyl-6-*t*-butylphenol). When present, the amount of the polymerization inhibitor used can be about 0.01 wt% to about 5 wt% of the mixture. Other conventional ingredients of compositions may be present in the pre-gel reaction mixture or added to the composition following the polymerization reaction.

The pre-gel reaction mixture may be spread or coated as a layer on a release liner, for example a siliconized release substrate such as silicone coated polyethylene terephthalate film, or other substrate prior to polymerization. Free radical polymerization may be initiated by, for example, heating the mixture when a thermal free radical polymerization initiator is present in the mixture, or exposing the mixture to actinic radiation when a photoinitiated free radical polymerization initiator is present in the mixture. Any convenient source or sources of actinic radiation providing wavelengths in the region of the spectrum that overlap the absorption bands of the photoinitiated free radical polymerization initiator can be used to activate polymerization. The radiation can be natural or artificial, monochromatic or polychromatic, incoherent or coherent, and for high efficiency should correspond closely in wavelengths to the absorption bands of the polymerization initiator. Conventional light sources include fluorescent lamps, mercury vapor lamps, metal additive lamps, and arc lamps. Useful lasers are those whose emissions fall within or overlap the absorption bands of the photoinitiated free radical polymerization initiator. Although, if desired, the mixture may be degassed before polymerization and/or the polymerization may be carried out under an inert atmosphere, it is not necessary to degas the mixture before polymerization or to carry out the polymerization under an inert atmosphere.

Following polymerization, the resulting composition may be transferred to a substrate. Alternatively, the composition may be adhered to a substrate, and the release liner left in place to protect the composition until it is ready for use.

While the above description contains many specifics, these specifics should not be construed as limitations on the scope of the disclosure herein but merely as exemplifications of particularly useful embodiments thereof. Those skilled in the art will envision many other possibilities within the scope and spirit of the disclosure as defined by the claims appended hereto.

## Claims

1. A gel composition comprising:
from substantially 33 wt% to substantially 68 wt% of a humectant or a mixture of humectants;
from substantially 0 wt% to substantially 8 wt% of an electrolyte or mixture of electrolytes;
from substantially 2 wt% to substantially 20 wt% of water;
from substantially 18 wt% to substantially 45 wt% of a copolymer comprising, in polymerized form, from substantially 80 mol% to substantially 95 mol% of a first monomer, in which the first monomer is a mixture of acrylic acid and one or more salts thereof, and from substantially 5 mol% to substantially 20 mol% of a second monomer, in which the second monomer is one or more monomers selected from CH₂=CHC(O)XR, in which X is O or NH and R is an unsubstituted or substituted alkyl group of from substantially 1 to substantially 5 carbon atoms;
in which the composition has a pH of substantially 7.0 or less; for use in the treatment of irritated or damaged dermal tissue.

2. A composition for use according to claim 1, wherein the second monomer in the gel comprises a salt of 2-acrylamido-2-methylpropane sulfonic acid, and the pH of the composition is from substantially 3.0 to substantially 6.5; and/or wherein the first monomer of the gel comprises a mixture of acrylic acid and sodium acrylate, and the second monomer comprises sodium 2-acrylamido-2-methylpropane sulfonate.

3. A composition for use according to claim 1 or claim 2, wherein the gel comprises from substantially 55 wt% to substantially 65 wt% of the humectant or the mixture of humectants and from substantially 20 wt% to substantially 40 wt% of the copolymer, and the copolymer comprises, in polymerized form, from substantially 85 mol% to substantially 95 mol% of the first monomer and from substantially 5 mol% to 15 mol% of the second monomer.

4. A composition for use according to any preceding claim, wherein the gel comprises from substantially 0.01 wt% to substantially 1.0 wt% of a crosslinking agent or a mixture of crosslinking agents.

5. A composition for useaccording to any preceding claim, wherein the dermal tissue comprises human breast tissue

6. A dermal patch for application to irritated or damaged dermal tissue comprising:
a substrate; and
a gel composition for placement adjacent the dermal tissue applied to the substrate, the gel composition comprising:
from substantially 33 wt% to substantially 68 wt% of a humectant or a mixture of humectants;
from substantially 0 wt% to substantially 8 wt% of an electrolyte or mixture of electrolytes;
from substantially 2 wt% to substantially 20 wt% of water;
from substantially 18 wt% to substantially 45 wt% of a copolymer comprising, in polymerized form, from substantially 80 mol% to substantially 95 mol% of a first monomer, in which the first monomer is a mixture of acrylic acid and one or more salts thereof, and from substantially 5 mol% to substantially 20 mol% of a second monomer, in which the second monomer is one or more monomers selected from CH₂=CHC(O)XR, in which X is O or NH and R is an unsubstituted or substituted alkyl group of from substantially 1 to substantially 5 carbon atoms; and
in which the composition has a pH of substantially 7.0 or less.

7. The dermal patch of claim 6 wherein the copolymer is either one of not crosslinked by a crosslinking agent, or crosslinked by a crosslinking agent.

8. The dermal patch of claim 6 or claim 7 wherein the second monomer is a salt of 2-acrylamido-2-methylpropane sulfonic acid, and the pH of the composition is from substantially 3.0 to substantially 6.5; and/or wherein the first monomer is a mixture of acrylic acid and sodium acrylate, and the second monomer is sodium 2-acrylamido-2-methylpropane sulfonate.

9. The dermal patch of any of claims 6-8 wherein the composition comprises from substantially 55 wt% to substantially 65 wt% of the humectant or the mixture of humectants and from substantially 20 wt% to substantially 40 wt% of the copolymer, and the copolymer comprises, in polymerized form, from substantially 85 mol% to substantially 95 mol% of the first monomer and from substantially 5 mol% to 15 mol% of the second monomer.

10. The dermal patch of any of claims 6-9 wherein the humectant is glycerol, sorbitol, or a mixture thereof, and the electrolyte is sodium chloride.

11. The dermal patch of any of claims 6-10 wherein the composition comprises from substantially 0.01 wt% to substantially 1.0 wt% of a crosslinking agent or a mixture of crosslinking agents.

12. The dermal patch of any of claims 6-11 including a release liner releasably attached to the gel composition, and/or further comprising a wicking layer, and/or further comprising an absorbent layer.

13. The dermal patch of any of claims 6-12, wherein the substrate has the shape of a cup to accommodate a mammalian breast.

14. A composition for use according to any of claims 1-5, wherein the treatment further comprises providing a substrate cover configured to retain the gel composition against the dermal tissue and prevent the gel composition from contacting another surface.

15. A gel composition comprising:
from substantially 33 wt% to substantially 68 wt% of a humectant or a mixture of humectants;
from substantially 2 wt% to substantially 20 wt% of water;
from substantially 18 wt% to substantially 45 wt% of a copolymer comprising, in polymerized form, from substantially 80 mol% to substantially 95 mol% of a first monomer, in which the first monomer is a mixture of acrylic acid and one or more salts thereof, and from substantially 5 mol% to substantially 20 mol% of a second monomer, in which the second monomer is one or more monomers selected from CH₂=CHC(O)XR, in which X is O or NH and R is an unsubstituted or substituted alkyl group of from substantially 1 to substantially 5 carbon atoms;
in which the composition has a pH of substantially 7.0 or less.
